# EUROPEAN PATENT APPLICATION

(11) **EP 4 549 848 A1**
(43) Date of publication of application: **07.05.2025**
(21) Application number: 23461674.6
(22) Date of filing: 30.10.2023
(51) Int. Cl.: F25D 3/14, A01N 1/02, A61J 1/16, B64C 39/02, B65D 81/18, F25D 11/00, F25D 17/04

(54) **A MULTI-CHAMBER CONTAINER, THE METHOD FOR CONTROLLING TEMPERATURE IN THE MULTI-CHAMBER CONTAINER AND THE USE OF THE MULTI-CHAMBER CONTAINER**

(71) Applicant: MedCool Sp. z o.o., 66-002 Zielona Gora (PL)
(72) Inventor: Klekiel, Tomasz, 65-012 Zielona Gora (PL); Noworol-Luft, Elzbieta, 26-630 Jedlnia-Letnisko (PL); Kieszek, Rafal, 02-765 Warszawa (PL)
(74) Representative: AOMB Polska Sp. z.o.o.

(57) **Abstract**

The present invention relates to the Field of containers For storing and transporting products sensitive to environmental conditions by using unmanned aerial systems. More specifically, the invention relates to boost the performance of air transport by using multi-chamber containers with the possibility of independent temperature control inside each storage chamber.

A multi-chamber container, especially For transporting medical materials with a drone, comprising a single temperature control system (1) and at least two storage chambers (2) For medical materials, characterized in that there are perforated barriers (3) between the storage chambers (2) and the chamber (4) with the temperature control system (1), said perforated barriers (3) configured For being movable between a First position, in which the perforations in the barriers (3) at least partially overlay one another and Form through openings, allowing circulation of air between the storage chambers (2) and the chamber (4) with the temperature control system (1) and a second position, in which the perforations in the barriers (3) miss one another, disabling circulation of air between the storage chambers (2) and the chamber (4) with the temperature control system (1).

## Description

### Technical Field

The present invention relates to the Field of containers For storing and transporting products sensitive to environmental conditions by using unmanned aerial systems. More specifically, the invention relates to boost the performance of air transport by using multi-chamber containers with the possibility of independent temperature control inside each storage chamber.

### Prior Art

The application of drones in medical logistics has garnered significant attention due to its potential to revolutionize healthcare delivery, particularly in emergency situations. Blood products, such as packed red blood cells, plasma, platelets, and other blood components, are crucial resources For patients in critical conditions, trauma cases, and during surgeries. Ensuring their timely and efficient delivery is vital For saving lives.

One of the key advantages of using drones For this purpose is their speed and agility. Drones can Fly directly to their destinations, bypassing traffic congestion and geographical barriers. In areas with limited or damaged infrastructure, such as remote rural regions or disaster-stricken areas, drones offer a reliable means of delivering blood products to patients in need. The ability to reach these locations quickly can be a matter of life and death, especially when time is of the essence.

Moreover, drones can operate beyond Fixed transportation routes, making them ideal For situations where conventional delivery methods may be impractical or unsafe. For example, in regions with challenging terrains like mountains, jungles, or flood-affected areas, drones can navigate efficiently and deliver critical medical supplies to isolated communities.

To maintain the integrity of blood products during transport, drones are equipped with advanced temperature control mechanisms. Blood components are highly sensitive to temperature Fluctuations, and maintaining the appropriate temperature range is crucial to preserve their quality and efficacy. Drones can be equipped with refrigeration units to ensure that blood products remain within the required temperature range throughout the journey.

To Further enhance the safety and reliability of blood product transportation, drones can be integrated into existing healthcare information systems. This allows medical professionals to track the location of the drone, monitor the status of the blood products in real-time, and receive alerts in case of any deviations or emergencies. This level of monitoring and coordination ensures that healthcare providers are well-inFormed and can respond swiftly to any unexpected events.

However, despite the numerous advantages, there are still challenges to overcome in implementing drone transportation For blood products. Ensuring regulatory compliance, airspace management, safety protocols, and public acceptance are critical aspects that require careful consideration.

The use of drones in the transportation of blood products presents a transformative opportunity For the medical Field. By leveraging the speed, Flexibility, and technology of drones, healthcare providers can enhance emergency response, save lives, and improve patient outcomes. Continued research, collaboration with regulatory bodies, and technological advancements will play a crucial role in unlocking the Full potential of drones in medical logistics, benefiting patients and healthcare systems worldwide.

In the state of the art, there are known solutions For the use of unmanned aerial vehicles in the transportation of blood and blood products.

Document TR202212262U discloses a system For organ transportation by unmanned aerial vehicle. The system comprises an unmanned aerial vehicle and a connected box. Within this box, several components are incorporated, including a temperature sensor a pressure module, a communication module, a dry ice bin, a vibration sensor, a GPS module, and a microcontroller card. The disclosed solution does not provide the possibility of a controlled independent multi-chamber cooling.

Document CN112265745B discloses a vehicle-mounted dual-purpose blood transport device with remote control capabilities. It includes a blood bag For carrying blood, a transport box For storing and transporting the blood bag, and a temperature control unit inside the transport box to regulate its operation and provide real-time transmission of blood bag inFormation. The blood transport logistics system connects to the transport box via the network and sends delivery orders to it. The blood bag is equipped with a shock unit For secure transportation. The disclosed solution still does not provide the possibility of independent multi-chamber cooling.

Document TR2021 08347A2 discloses a thermoelectric biomaterial carry bag which ensures safe thermal conditions and adheres to hygienic requirements. The bag is designed to Facilitate both human-powered transportation and transportation via unmanned aerial vehicles, however it still does not provide the possibility of independent multi-chamber cooling.

Document US20220174943A1 discloses a method of storing biomaterials, blood, blood products, vaccines, and organs through a specialized system designed For organ and Fluid preservation and transportation. This system comprises various components, including a docking system, a container system, electronics, and a heating/cooling module. The container system maintains consistent environmental conditions when closed and it can be attached to the docking system, where the docking system regulates the temperature by either heating or cooling the container system. The system uses a Peltier cell For temperature control. This solution does not provide the possibility of independent multi-chamber cooling.

The utility model CN216862420U pertains to the medical instruments Field and presents a blood collection container. The container includes a main body, a platelet storage compartment, a plasma storage compartment, and a red suspension storage compartment, all situated within the main body's inner cavity. The storage compartments incorporate a constant-temperature control mechanism. The disclosed solution does not provide For use in transportation by unmanned aerial vehicles.

### Summary of the Invention

According to the invention a multi-chamber container, especially For transporting medical materials with a drone, comprising a single temperature control system and at least two storage chambers For medical materials, comprising perforated barriers between the storage chambers and the chamber with the temperature control system, said perforated barriers configured For being movable between a First position, in which the perforations in the barriers at least partially overlay one another and Form through openings, allowing circulation of air between the storage chambers and the chamber with the temperature control system and a second position, in which the perforations in the barriers miss one another, disabling circulation of air between the storage chambers and the chamber with the temperature control system.

Preferably the multi-chamber container Further comprises a perforated separator between the perforated barriers wherein the perforated separator is movable between a First position, in which the perforations in the barriers and in the separator at least partially overlay one another and Form through openings, allowing circulation of air between the storage chambers and the chamber with the temperature control system and a second position, in which the perforations in the barriers and in the separator miss one another, disabling circulation of air between the storage chambers and the chamber with the temperature control system.

Preferably the multi-chamber container comprises at least one Forcing air movement device, especially a Fan.

Preferably the multi-chamber container comprises means For mounting it to or in an unmanned aerial vehicle, preferable a drone.

PreFerably the multi-chamber container contains a heat insulation of at least one layer of any insulating material between the inner space and the body of the multi-chamber container, wherein the heat insulation has a thickness of at least 4 cm and a conductivity coefficient of less than 0.03 W/m*K.

PreFerably there is a thermal insulation Foil between the body of the multi-chamber container and subsequent heat insulation layers.

Preferably the power system of the multi-chamber container comprises a battery to provide independence From external power sources.

Preferably the multi-chamber container Further comprises a monitoring system that comprises sensors, software and communication technologies to track the status of the device.

Another aspect of the invention is a method For controlling temperature in the multi-chamber container wherein the temperature in controlled by moving said perforated barriers and optionally said perforated separator between said First position and said second position.

PreFerably the temperature in each said storage chamber is controlled independently.

Preferably the temperature is controlled in the range From -20 to 20 °C.

Yet another aspect of the invention is a use of the multi-chamber container For the transportation of blood, blood products, organs, and other medical materials by an unmanned aerial vehicle, especially a drone.

The disclosed solution describes a device designed For the transportation of blood products and related biomaterials, capable of collaborating with unmanned aerial systems used in medical transport. The device Features a multi-compartment temperature regulating system with real-time environmental monitoring For each chamber, ensuring optimal storage conditions and minimizing contamination risks.

The device's subsystems are powered by rechargeable batteries but can also utilize power From the drone's onboard system through an automated energy management system. Additionally, there is an option For external power sources, such as an extra battery, or direct connection to electrical mains (230V/50Hz) or any DC power source within the range of 12V-80V with sufficient current output. This provides Flexibility and reliability in various power supply scenarios.

The device is equipped with a monitoring and data logging subsystem For cargo parameters during transportation, enabling real-time data collection and recording. Furthermore, it integrates a data transmission subsystem compatible with the telemetry BSP system, specifically tailored For the transportation of biological materials. This Facilitates real-time tracking and monitoring of the transported materials, enhancing the overall efficiency and safety of the process.

The device includes a communication interface For direct visualization of its status, allowing For convenient control and monitoring. Additionally, a wireless information system For mobile devices is implemented, providing remote access to data and transportation information, which proves particularly valuable in critical situations requiring prompt responses.

As a result, this advanced transport device ensures a secure and efficient means of transporting blood products and other biomaterials, significantly impacting life-saving efforts and improving the quality of healthcare services.

The proposed solution makes it possible to realize a multi-chamber temperature stabilization process in which one system will be able to regulate the temperature independently in individual storage compartments and there will be different temperatures in them.

A drone blood transport container used in medical or life-saving applications should meet a number of specific requirements and have some important Features. The container as well as the cooling system are designed to withstand vibrations and shocks that can occur during drone Flight. Blood products are Fragile and sensitive to damage. Moreover The container should be equipped with thermal insulation and protection From rain, moisture and other weather conditions to protect the contents. In addition, the container must be tailored to the specific requirements and regulations of the country.

### Brief Description of the Drawings

In order to provide more complete understanding of the claimed invention and advantages thereof, the Following description provides an explanation of possible exemplary embodiments thereof with a reference to Figures of the appended drawings, wherein identical designations denote identical parts, and which illustrate the Following:
Fig. 1 shows a diagram of the operation of the cooling system For a two-chamber container (prior art);
Fig. 2 shows a diagram of the operation of the three-chamber container (prior art);
Fig. 3 shows a diagram of an arrangement of chambers in a temperature control device;
Fig 4 shows an alternative compact solution in which the drone body and container Form a cohesive entity and can be designed together;
Fig. 5 shows selected concepts of device structure;
Fig. 6 shows diagram of operation of the insulating barrier.

### Main designations:

1. Compressor temperature control system;
2. The storage chambers;
3. Perforated barriers;
4. Chamber with the temperature control system;
5. Perforated separator;
6. Fan.

### Preferred Embodiments of the Invention

According to the invention a multi-chamber container comprising a single temperature control system 1, in which the temperature in the storage chambers 2 is regulated independently through a system of perforated barriers 3 between storage chambers 2 and the chamber 4 with the temperature control system. In particular the temperature control system 1 is in the Form of a compressor system, Peltier device, ice pack, dry ice and other methods that give the possibility of controlling the temperature.

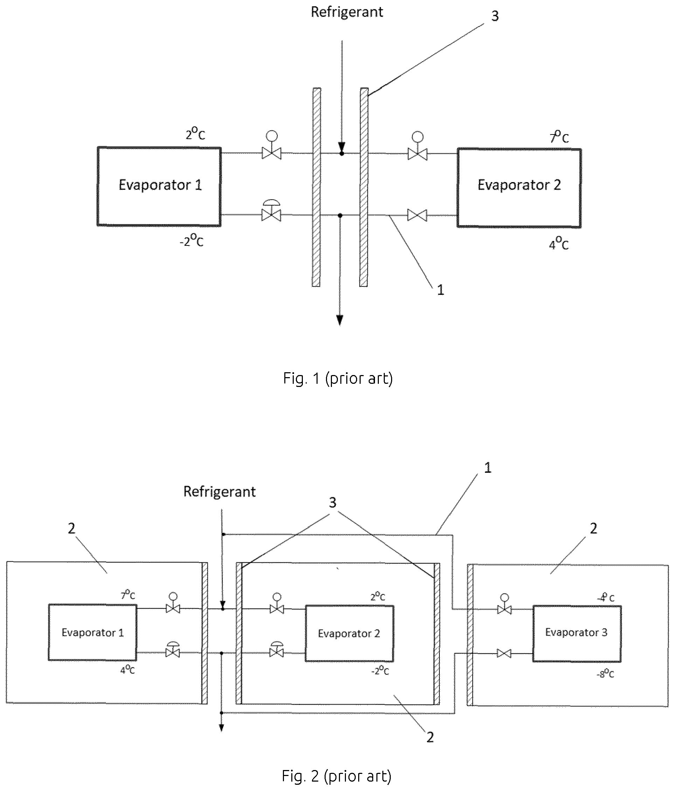

The solution of the compressor temperature control system comprising a compressor, a condenser, an evaporator and throttling system can be realized in the conFiguration according to Fig. 1 or Fig. 2 in which the system includes an evaporator unit independently operating several storing chambers of different temperatures. The system of operation of the independent temperature control system For three chambers using one compressor and one condenser is presented in Fig. 2.

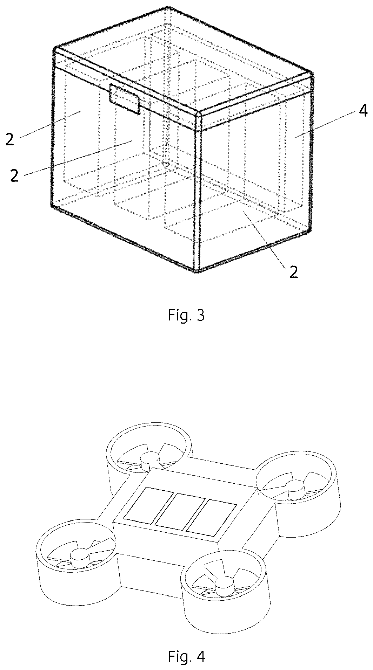

The possibility of placing evaporator elements in the chambers is shown schematically in Fig. 3. By controlling the regulating valves, it is possible to achieve a temperature close to the set temperature value in a given chamber.

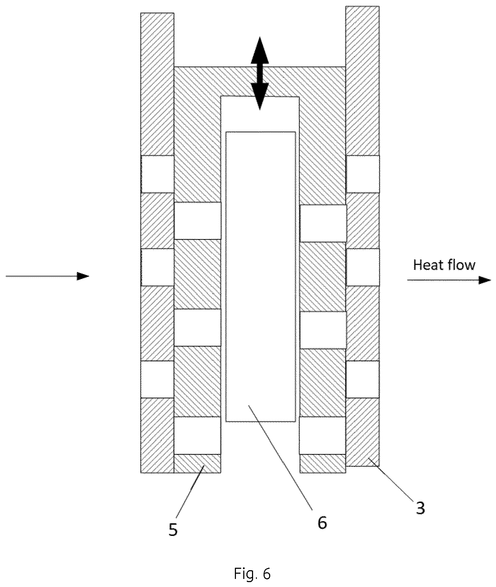

The multi-chamber container preferably comprises additional perforated separator 5. Fig. 6 shows the idea of regulating and Forcing heat Flow between chambers. The direction of heat Flow is Forced by air movement caused by a Fan 6 located inside the perforated barrier. The barrier preferably comprises the separator 5 moving vertically. The perforated barriers 3 together with the perforated separator allow the openings to be covered or uncovered, enabling or preventing the exchange of heat by convection. By adjusting the thickness of the barriers 3, adopting the appropriate number of openings and by adjusting the operation of the Fan 6, it is possible to control the Flow of heat between the chambers.

The position of Fan 6 in the separator 5 shown in Fig. 6 increases the insulation conditions by increasing the thickness of the wall separating the heat sources and the storage chamber 2, and at the same time is optimal in terms of air transport and space occupied. However, the indicated position of the Fan is not limited to this location and can be located elsewhere.

Since the multi-chamber container is designed to be transported by drones, it also includes the appropriate mounting means. The method of connecting the container is based on the structure and design of the specific drone model. In the case of a cargo hold, the container will be inserted and secured with a Flap or straps. As shown in Fig. 4, the proposed solution can also be used in a compact solution in which the body of the drone and the container Form a coherent whole and can be designed together. In some drones, the container can be suspended under the drone using special solutions such as cables and brackets. Mounting on top possible in some drones allows the container to be mounted directly on top of the drone. This may involve special handles or mounting systems. The bottom of the container housing should be equipped with a suitable catch. Integrated storage compartments are Found in drones, especially those intended For cargo delivery. In this case, the container is placed in a special space inside the drone, which is secured and provides stability during Flight. Some drones have special modules or accessories available that can be mounted to carry specific types of cargo. Preferably the container will be insulated with a material with a thickness of at least 4 cm and a conductivity coefficient of no more than 0.025 W/m*K. Such materials are well-known insulation materials, i.e. mineral wool, polyurethane Foams of various densities, polystyrene Foam, aerogels, etc. A thermal insulation Film used as a rescue thermal blanket to reflect thermal radiation waves and increase insulation efficiency will be placed between the casing and successive layers of insulation. The realization of the device regulating the temperature in the container according to the invention can be carried out in many variants through the appropriate selection of components including, First of all, the component that realizes the heat exchange between the transport chambers and the environment, appropriate insulation, the mechanism For accessing the individual chambers and an adequate control system.

In the case of the designed temperature control system, one of the main assumptions is that the device is intended to be transported by drones, and therefore such limitations as geometric dimension weight and limited access to power and electricity result From this, so when considering the configuration of the cooling system, considerable attention should be paid to the appropriate design solution of the insulation which will adequately protect the transported object at the assumed time regime. Sufficiently high insulation efficiency means that the need to consume energy For temperature maintenance is minimized, as less thermal energy is dissipatively lost.

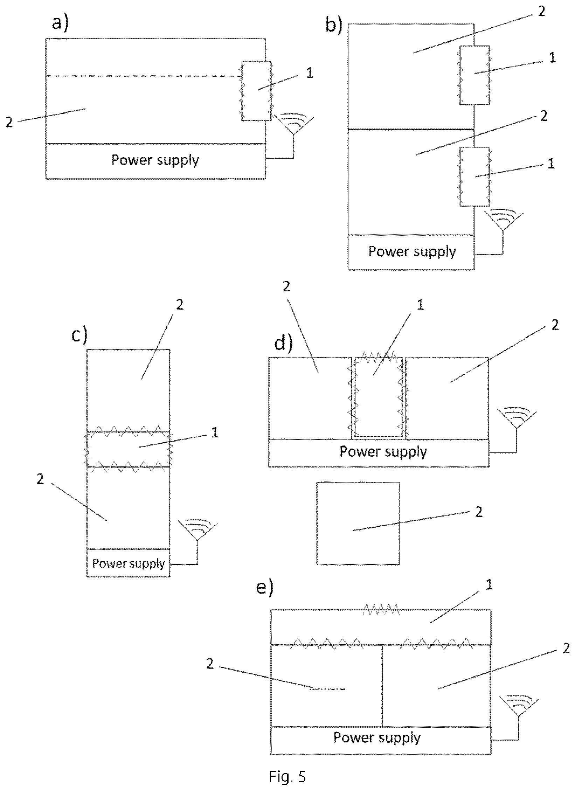

Figure 5 shows the analyzed structures of the cooling device. Fig. 5a shows a typical structure of a cooling system in which the chamber is placed in a central point while the temperature control system is located on one of the walls of the cargo space. Given the need For Full autonomy of the device, space should be provided For battery power cells. In this arrangement, it is envisioned that the power cells will be placed at the bottom of the object to lower the center of gravity and allow easy replacement of cells, such as swapping to charged cells.

In turn, Fig. 5b shows a solution in which the chambers are placed independently one above the other, equipping each of them with a separate temperature control system. Thus, in such an arrangement, each chamber can be independent of the others. The structure of the device can be modular which allows any conFiguration. However, the biggest drawback of such a solution is the need For multiple temperature control systems, which adversely affects the weight of the whole. Fig. 5c shows the conFiguration of the device, in which the temperature control system divides the storage compartments. Such a solution allows to achieve autonomy of each chamber which is conducive to maintaining temperature differences in each chamber, but such a solution can be useful For a small number of chambers. Similarly, the structure of the device according to the diagram in Fig. 5d limits the possibility of cascading chambers of greater numbers. The structure shown in Fig. 5e seems to be the most versatile, as it resembles bus structures. In this arrangement, the bus is a temperature control device to which any number of chambers, although limited due to overall dimensions, can be connected.

The temperature control system is designed to maintain the temperature preferably in the range of From -20 to 20 °C. Due to the Forced air movement inside the storage compartments, the temperature is measured at more than one place in the upper part of the storage compartment and the lower part. Independently control requires measuring the temperature of the heat/cold source. In the preferred embodiment the power system comprises batteries to provide independence From external power sources. Moreover it comprises a monitoring system that comprises sensors, software and communication technologies to track the status of the device.

## Claims

1. A multi-chamber container, especially For transporting medical materials with a drone, comprising a single temperature control system (1) and at least two storage chambers (2) For medical materials, **characterized in that** there are perforated barriers (3) between the storage chambers (2) and the chamber (4) with the temperature control system (1), said perforated barriers (3) configured For being movable between a First position, in which the perforations in the barriers (3) at least partially overlay one another and Form through openings, allowing circulation of air between the storage chambers (2) and the chamber (4) with the temperature control system (1) and a second position, in which the perforations in the barriers (3) miss one another, disabling circulation of air between the storage chambers (2) and the chamber (4) with the temperature control system (1).

2. The multi-chamber container according to claim 1, **characterized in that** it Further comprises a perforated separator (5) between the perforated barriers (3) wherein the perforated separator (5) is movable between a First position, in which the perforations in the barriers (3) and in the separator (5) at least partially overlay one another and Form through openings, allowing circulation of air between the storage chambers (2) and the chamber (4) with the temperature control system (1) and a second position, in which the perforations in the barriers (3) and in the separator (5) miss one another, disabling circulation of air between the storage chambers (2) and the chamber (4) with the temperature control system (1).

3. The multi-chamber container according to claim 1 or 2, **characterized in that** it comprises at least one Forcing air movement device, especially a Fan (6).

4. The multi-chamber container according to any one of claims 1 to 3, **characterized in that** it comprises means For mounting it to or in an unmanned aerial vehicle, preFerable a drone.

5. The multi-chamber container according to any one of claims 1 to 4, **characterized in that** it contains a heat insulation of at least one layer of any insulating material between the inner space and the body of the multi-chamber container, wherein the heat insulation has a thickness of at least 4 cm and a conductivity coefficient of less than 0.03 W/m*K.

6. The multi-chamber container according to any one of claims 1 to 5, **characterized in that** between the body of the multi-chamber container and subsequent heat insulation layers there is a thermal insulation Foil.

7. The multi-chamber container according to any one of claims 1 to 6, **characterized in that** its power system comprises a battery to provide independence From external power sources.

8. The multi-chamber container according to any one of claims 1 to 7, **characterized in that** it Further comprises a monitoring system that comprises sensors, software and communication technologies to track the status of the device.

9. A method For controlling temperature in the multi-chamber container of any of claims 1 to 8, **characterized in that** the temperature in controlled by moving said perforated barriers (3) and optionallysaid perforated separator (5) between said First position and said second position.

10. The method according to claim 9, **characterized in that** the temperature in each said storage chamber (2) is controlled independently.

11. The method according to claim 9 or 10, **characterized in that** the temperature is controlled in the range From -20 to 20 °C.

12. A use of the multi-chamber container of any of claims 1 to 8 For the transportation of blood, blood products, organs, and other medical materials by an unmanned aerial vehicle, especially a drone.
